# EUROPEAN PATENT SPECIFICATION

(11) **EP 1 511 761 B1**
(45) Date of publication and mention of the grant of the patent: **02.08.2006**
(21) Application number: 04759123.5
(22) Date of filing: 05.04.2004
(51) Int. Cl.: C07K 1/06, C07K 7/56, C07K 14/655

(54) **PROCESS FOR PRODUCTION OF CYCLIC PEPTIDES**
VERFAHREN ZUR HERSTELLUNG CYCLISCHER PEPTIDE
PROCEDE RELATIF A L'ELABORATION DE PEPTIDES CYCLIQUES

(30) Priority: 07.04.2003 US 461222 P
(43) Date of publication of application: 09.03.2005
(73) Proprietor: Novetide Ltd., Haifa Bay 226111 (IL)
(72) Inventor: TOVI, Avi, 20104 Zurit (IL); EIDELMAN, Chaim, 25167 Tal-El (IL); SHUSHAN, Shimon, 21926 Karmiel (IL); ELSTER, Shai, 32811 Haifa (IL); ALON, Hagi, 25125 Kibbuz Yehiam (IL)
(74) Representative: Nachshen, Neil Jacob
(86) International application number: PCT/US2004/010426
(87) International publication number: WO 2004/092202

(56) References cited:
- EP-A- 0 953 577
- WO-A-01/05757
- WO-A-99/14238
- US-A- 4 351 764
- US-A- 5 500 413
- US-A- 5 686 570

## Description

### Priority

This application claims the benefit of U.S. provisional application Serial No. 60/461,222, filed April 7, 2003, the content of which is incorporated herein.

### Field of the Invention

The present invention encompasses processes for the preparation and purification of cyclic peptides.

### Background of the Invention

Somatostatin is known to possess a very broad therapeutic potential and can be administered in a wide variety of clinical applications. The mean half-life in plasma of somatostatin is extremely short, therefore reducing the potential number of possible applications of this reagent. Research was carried out with the aim of developing analogs of somatostatin which exhibited greater stability and efficacy. One series of compounds which were evaluated as potentially useful somatostatin analogs were cyclic octapeptides. Evaluation of the cyclic octapeptide, octreotide, demonstrated that the compound had excellent biological activity both in vitro and in vivo (Pless J., Metabolism 41, 5-6 (1992)). Octreotide has the following basic formula: 
D-Phe—Cys—Phe—D-Trp—Lys—Thr—Cys—Thr-ol
(SEQ. ID NO. 1)
 wherein the sulfur atoms of the Cys residues at the positions 2 and 7 are bounded by a disulfide bridge. The carboxylic group of the C-terminal amino acid, threonine (Thr), is reduced to the alcohol Thr-ol (threoninol) residue.

The presence of D-phenylalanine (D-Phe) at the N-terminal end and of an amino alcohol at the C-terminal end, along with the D-tryptophan (D-Trp) residue and the disulfide bridge, make the molecule very resistant to metabolic degradation. Octreotide permits a 24 hour incubation in aggressive mediums, such as gastric juices or in intestinal mucosa.

Octreotide inhibits growth hormone for a lengthy period, inhibits the secretion of glucagon to a lesser degree, and inhibits insulin secretion only in a transient manner. Thus, octreotide is selective more than other somatostatin analogues in regulating the levels of growth hormone in the body and therefore, presently is indicated in acromegaly to control and reduce the plasma levels of such hormone. Also, octreotide is useful in the treatment of cellular alterations of gastroenteropancreatic endocrine origin and of certain types of tumors.

The synthesis of octreotide and its derivatives has been described by two general synthetic methods. The first method is a solution phase procedure, based on fragment condensation, as described by Bauer *et al.* European Patent Application No. 29,579 (1981) and U.S. Patent No. 4,395,403. The process generally comprises removing a protecting group from a protected hexapeptide residue; linking together two peptide units by an amide bond, wherein one comprises a hexapeptide residue; converting a functional group at the N- or C-terminal end of the resulting polypeptide; and oxidizing the polypeptide. The process involves a time-consuming, multi-step synthesis, and presents additional problems during the separation of octreotide from the reaction mixtures because all the synthetic steps are carried out in solution phase.

The second method for the synthesis of octreotide synthesizes the entire peptide chain using solid phase peptide synthesis, starting the synthesis at the threoninol residue. This method requires that the threoninol residue be protected.

The second synthetic process, uses an aminomethyl resin upon which the threoninol residue is incorporated with the two alcohol functions protected in acetal form. Mergler *et al.,* "Peptides: Chemistry and Biology," *Proceedings of the 12th American Peptide Symposium,* Poster 292 Presentation (Smith, J. A. and Rivier J. E., Eds ESCOM, Leiden) (1991). The synthesis is carried out following an Fmoc/t-Bu protection scheme; forming the disulfide bridge on a resin by oxidation of the thiol groups of the previously deprotected cysteine residues; and releasing and deprotecting the peptide with a 20% mixture of TFA/DCM.

Alsina *et al.* described the incorporation of a threoninol residue on active carbonate resins wherein the amino group protected by a Boc group and the side chain protected by a Bzl group. Alsina *et al., Tetrahedron Letters,* 38, 883-886 (1997). Thereafter, the synthesis continued using a Boc/Bzl strategy. Formation of the disulfide bridge was carried out directly on resin using iodine, and the peptide was cleaved from the resin and its side chain protecting groups were simultaneously removed with HF/anisole (9/1). At a final stage the formyl group was removed with a piperidine/DMF solution. Neugebauer *et al.* described a linear synthesis with a yield of only 7%. Neugebauer *et al.,* PEPTIDES: CHEMISTRY, STRUCTURE AND BIOLOGY, p. 1017 (Marshal G. R. and Rivier J. E., Eds ESCOM, Leiden, 1990).

Edwards *et al.* disclosed a solid-phase type approximation by the stepwise synthesis on a resin of the peptide D-Phe-Cys(Acm)-Phe-D-Trp(Boc)-Lys(Boc)-Thr(t-Bu)-Cys(Acm)-HMP-resin (SEQ. ID NO. 1). Edwards *et al., J. Med Chem.* 37, 3749-3757 (1994). Subsequently, the disulfide was prepared on the resin, and the resultant product released from the resin by means of aminolysis with threoninol. The total yield reported was only 14%.

Arano *et al.* carried out another solid phase method for DTPA-octreotide. Arano *et al., Bioconjugate Chem.,* 8,442-446 (1997). The iodine oxidation of the DTPA-peptide produced DTPA-D-Phe^{*I*}-octreotide in overall 31.8% yield based on the starting Fmoc-Thr(tBu)-ol-resin.

Wu *et al.* developed a synthetic method for octreotide, wherein the disulfide bond was formed by oxidation using a dilute solution of octreotide with air during 48 hours. Wu *et al., Tetrahedron Letters,* 39, 1783-1784 (1998). Lee *et al.* recently carried out a new method to anchor Thr(ol) (or Thr-ol) to a solid phase synthesis resin for preparation of octreotide. See, U.S. Patent No. 5,889,146. Fmoc-Thr(ol)-terephthal-acetal was loaded onto the resin and after construction of peptide chains using Fmoc chemistry, the cyclization of the peptide was obtained on resin by oxidation with iodine. The cleavage of peptide-resin with trifluoroacetic acid, produced octreotide with an overall yield of >70% from the starting Fmoc-Thr(ol)-terephthal-acetal-resin. All of these procedures completed the cyclization of the octreotide either on totally deprotected peptide or on the resin.

Further cyclic, bridge cyclic, and straight-chain somatostatin analogues and methods for their preparation are described in U.S. Patent Nos. 4,310,518 and 4,235,886; European Patent Specifications EP-A-1295; 70,021; 113,209; 215,171; 203,031; 214,872; and 143,307; and Belgian Patent Specification BE-A-900,089.

### Summary of the Invention

The present invention encompasses processes for the preparation and purification of cyclic peptides. In one embodiment, the processes comprise (a) providing a protected linear peptide, wherein the peptide contains at least two protected thiol-containing residues, of which at least one is protected with an orthogonal protecting group; (b) reacting the protected linear peptide with an acidic composition to produce a semi-protected linear peptide protected with the orthogonal protecting group on one of the thiol-containing residues; (c) purifying the semi-protected linear peptide by chromatography; (d) treating the purified semi-protected linear peptide obtained in step (c) with an oxidizing agent to produce an unprotected cyclic peptide; and (e) purifying the unprotected cyclic peptide by chromatography. In one embodiment, the process further comprises neutralizing excess oxidizing agent after step (d). In another embodiment, the oxidizing agent may be iodine.

In a preferred embodiment of the invention, the cyclic peptide is selected from the group consisting of somatostatin analogues, vasopressin related peptides, α-atrial natriuretic factors/peptides (ANF/ANP), calcitonins and other disulfide containing peptides. In a more preferred embodiment, the cyclic peptide is octreotide, calcitonin (salmon), desmopressin, oxytocin, nesiritide, or eptifibatide.

In yet another embodiment of the invention, the linear peptide of step (a) is attached to a resin or in solution.

In another embodiment of the invention, the orthogonal protecting group is acetamidomethyl, benzyl, 4-methoxybenzyl, tert-butyl, trimethylacetamidomethyl, phenylacetamidomethyl, or tert-butylmercapto. Preferably, the orthogonal group is a non-acid labile protecting group such as acetamidomethyl (ACM).

One embodiment of the invention encompasses a process for preparing a cyclic peptide having a purity of at least about 98.5% by HPLC and preferably, at least about 99% by HPLC. Another embodiment of the invention is the preparation of a cyclic peptide at high purity, by a process wherein the reaction product upon cleavage from a support resin, when attached to a support resin, is partially deprotected and carries at least one protecting group attached to a thiol-containing residue.

Another embodiment of the invention encompasses a process employing Acm as a protecting group for cysteine residues.

### Detailed Description of the Invention

The invention encompasses methods for preparing polypeptides. More specifically, the invention encompasses methods for preparing cyclic polypeptides wherein at least one protecting group attached to thiol-containing residues, such as cysteine residues, is not cleaved or deprotected from the thiol-containing residues under the conditions required for cleavage other protecting groups. Thus, the peptide chain is not completely deprotected, but carries at least one protecting group attached to a thiol-containing residue. Specifically, the invention encompasses processes for preparing cyclic peptides selected from the group consisting of somatostatin analogues, vasopressin related peptides, α-atrial natriuretic factors/peptides (ANF/ANP), calcitonins, and other disulfide containing peptides. More specifically, the cyclic peptide is selected from the group consisting of octreotide, calcitonin (salmon), desmopressin, oxytocin, nesiritide, and eptifibatide.
Nesiritide has the following peptide sequence:

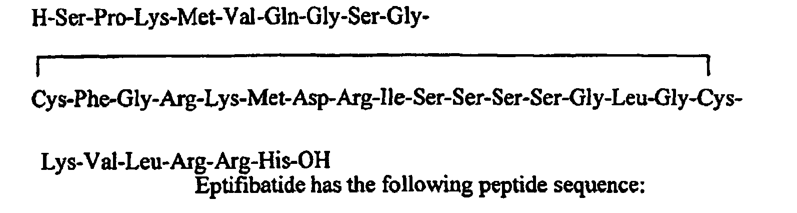
H-Ser-Pro-Lys-Met-Val-Gln-Gly-Ser-Gly-
Cys-Phe-Gly-Arg-Lys-Met-Asp-Arg-Ile-Ser-Ser-Ser-Ser-Gly-Leu-Gly-Cys-
Lys-Val-Leu-Arg-Arg-His-OH
Eptifibatide has the following peptide sequence:

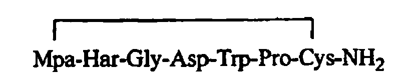
Mpa-Har-Gly-Asp-Trp-Pro-Cys-NH2

The reaction product may be purified to obtain a cyclic peptide of high purity. As used herein, the term "high purity" refers to a composition comprises at least about 98.5% as determined by HPLC, and preferably at least about 99% as determined by HPLC. As used herein, the term "cyclic peptide" refers to a peptide containing at least two (2) thiol-containing residues connected by a disulfide bridge.

The synthesis of the semi-protected peptide may be performed by known methods for peptide synthesis, for example, on a solid support or in solution, among others. The process of the invention for preparing cyclic peptides is a process wherein at least one thiol-containing residue of the starting material is protected by an orthogonal protecting group that is not cleaved or deprotected from a thiol-containing residue under the conditions required for the cleavage of other protecting groups or cleavage of the peptide from the resin. As used herein, the term "orthogonal protecting group" refers to a protecting group that is chemically resistant under one set of selected conditions, but is liable under another set of conditions. Orthogonal protecting groups include, but are not limited to, at least one of acetamidomethyl (Acm), benzyl (Bzl), 4-methoxybenzyl (Mob), tert-butyl (Tbu or t-Bu), trimethylacetamidomethyl (Tacm), phenylacetamidomethyl(Phacm), or tert-butylmercapto (StBu). Preferably, the orthogonal protecting group is a non-acid labile group such as acetamidomethyl. Thus, in the process of the present invention, the peptide chain is not completely deprotected after deprotection or cleavage, but carries at least one protecting group attached to a thiol-containing residue (a semi-protected polypeptide). Optionally, the semi-protected polypeptide may be purified using any suitable methods. Subsequently, the remaining protecting groups on the semi-protected polypeptide are removed and a disulfide bridge formed to obtain a cyclic peptide using any conventional method. For example, one method includes, but is not limited to, thiol oxidation by an oxidizing agent such as iodine. The cyclic peptide is purified by suitable methods to obtain a high purity cyclic peptide. Optionally, if present, excess oxidizing agent can be neutralized prior to purification. Preferably, purification is carried out using HPLC.

For the purpose of clarity and as an aid in the understanding of the invention, as disclosed and claimed herein, the following terms and abbreviations are defined below:
- TIS -: Triisopropylsilane
- Trt -: trityl
- Mpa -: mercaptopropionic acid.

On a polypeptide, one or more of the thiol-containing residues are protected using an orthogonal protecting group such as acetamidomethyl (Acm). When Acm is used as a protecting group of the thiol-containing residue, acidolytic cleavage of the peptide will yield a peptide sequence carrying one Acm group. For example, in the preparation of octreotide, if Acm is used as a protecting group of a first thiol-containing residue, *e.g.,* the cysteine residue attached to Thr-ol, acidolytic cleavage of the peptide will yield a peptide sequence carrying one Acm group, for example, D-Phe-Cys-Phe-D-Trp- Lys-Thr-Cys(Acm)-Thr(ol). Peptides may be characterized by a specific profile of related impurities observed by HPLC analysis. Some of these impurities are easily separated from the main peak whereas others elute more closely to the main peak. Coarse purification by preparative HPLC produces the partially protected peptide at a purity of at least about 95%, preferably at least about 98.5%, and most preferably, at least about 99% as determined by HPLC, and at a concentration of about 0.1 g/L to about 10 g/L. Addition of an approximately equivalent amount of an oxidizing agent, such as iodine, results in deprotection of the orthogonal protecting group and simultaneous cyclization of the molecule through a disulfide bridge. An evaluation of the chromatographic profile at this stage shows clearly that some of the impurities that were previously eluted close to the main peak are now distinct from the product peak. Thus, the product may be easily purified a second time by methods such as HPLC or other known methods to obtain a purified peptide such as octreotide in high purity. By these means, high purity product is easily obtained in high yield without need for several recycling cycles that require large volumes of solvents, long operation time, and results in a lower purity and lower yield of the final product in comparison to conventional methods currently used.

The process for peptide synthesis according to one embodiment of the invention is carried out by the following scheme:
a) Providing a peptide sequence containing at least two protected thiol-containing residues, of which at least one is protected with a non-acid labile protecting group;
b) Cleavage of acid sensitive protecting groups using an acidic composition comprising various scavengers to form a semi-protected linear peptide;
c) Coarse purification of the semi-protected linear peptide by suitable chromatographic method;
d) Deprotection of the residual non-acid labile protecting group of the semi-protected linear peptide, and cyclization of the linear peptide via formation of disulfide bond by suitable method to form a crude cyclic peptide solution; and
e) Purification of crude cyclic peptide solution by suitable separation method to obtain a cyclic peptide product.

The resulting peptide solution may be dried to obtain a dry cyclic peptide product. When the peptide is synthesized on a solid phase, such as a resin and the orthogonal protecting groups are non-acid labile groups, the acidic composition used for deprotection of acid sensitive protecting groups also cleaves the resulting semi-protected peptide from the resin.

One particular example of the process for peptide synthesis described above comprises the steps of:
1. Preparation of a protected peptide sequence on a resin. For example, in the preparation of octreotide, a suitable starting material may be Thr(ol)(t-Bu)-2-chlorotrityl resin. Thereafter, a linear peptide is synthesized by a cycle of repetitive operations in the following order.
1.1. Coupling of a suitable Fmoc-protected amino acid with a suitable coupling reagent to the terminal amino group residue attached to the resin to form a Fmoc-protected peptide fragment attached to the resin.
1.2. Washing the product of step 1.1 with at least one solvent to remove all soluble compounds from the resin.
1.3. Deprotection of the Fmoc group.
1.4. Washing with at least one solvent to remove all soluble compounds from the resin.
1.5. Adding additional amino acid residues according to the required sequence, followed by washing with at least one solvent and deprotection of the Fmoc group as necessary.
1.6. Coupling of the last amino acid D-Phe (N-protected).
1.7. Washing and drying of the peptide-resin.
2. Cleavage of the linear peptide intermediate (partially protected) from the resin and simultaneous deprotection of the acid sensitive protecting groups using an acidic composition comprising trifluomacetic acid (TFA) and various scavengers to form a semi-protected linear peptide.
3. Coarse purification of the semi-protected linear peptide by suitable chromatographic method.
4. Deprotection of the residual protecting group of the semi-protected linear peptide.
5. Cyclization of the linear peptide via formation of disulfide bond by suitable method to form a crude cyclic peptide solution.
6. Purification of crude cyclic peptide solution by suitable separation method.

When synthesizing the peptide on a solid support, suitable resins for use in the process include, but are not limited to, chlorotrityl resins. Suitable coupling agents include, but are not limited to, 2-(1H-benzotriawle-1-yl)-1,1,3,3-tetramethyluronium tetrafluoroborate (TBTU). Suitable solvents for use in the washing steps of the process include, but are not limited to, dimethylformamide (DMF), dichloromethane (DCM), methanol (MeOH), or isopropanol (IPA). Suitable protecting groups for the terminal amino acid residue include, but are not limited to, 9-fluorenylmethoxycarbonyl (Fmoc) or Boc. Suitable protecting groups for the cysteine residues include Acm. The terminal amino acid residue protecting group is removed by any known method, such as reaction with piperidine solution in DMF. Although one of ordinary skill in the art may substitute the reagents with other suitable reagents.

Cleavage of the partially protected linear intermediate from the resin support may be effected by addition of an acidic composition. The acidic composition is preferably based on an acidic material such as TFA, and contains scavenger reagents including, but not limited to, ethanedithiol (EDT) and water. The relative ratio of acidic material to scavenger to water may be from about 85% to about 99% acidic material, from about 0.1% to about 15% scavenger, and from about 0.1% to about 15% water by weight. A preferred acidic composition comprises about 95% TFA, about 2.5% EDT, and about 2.5% water.

The crude peptide product may be purified by any known method. Preferably, the peptide is purified using HPLC on a reverse phase (RP) column. The resulting purified product is dried and may be lyophilized.

Particular embodiments of the process of the present invention include, but are not limited to, the synthesis of octreotide, eptifibatide, desmopressin, and calcitonin salmon, as exemplified below.

One embodiment of the present invention provides a process for preparing octreotide comprising the steps of:
a) providing a protected linear peptide having the formula X-D-Phe-Cys(X)-Phe-D-Trp-Lys(X)-Thr(X)-Cys(Acm)-Tbr(X)-ol wherein X is same or different protecting groups;
b) reacting the linear peptide of step (a) with an acidic composition to produce D-Phe-Cys-Phe-D-Trp-Lys-Thr-Cys(Acm)-Thr-ol;
c) purifying the product of step (b) by HPLC;
d) treating the resulting linear peptide of step (c) with an oxidizing agent to produce (2,7 cyclic) D-Phe-Cys-Phe-D-Trp-Lys-Thr-Cys-Thr-ol and
e) purifying the product of step (d) by HPLC.

Another embodiment of the present invention provides a process for preparing eptifibatide comprising the steps of:
a) providing a protected linear peptide having the formula Mpa(X)-Har(X)-Gly-Asp(X)-Trp-Pro-Cys(Acm) wherein X is same or different protecting groups;
b) reacting the linear peptide of step (a) with an acidic composition to produce Mpa-Har-Gly-Asp-Trp-Pro-Cys(Acm)-NH₂
c) purifying the product of step (b) by HPLC;
d) treating the resulting linear peptide of step (c) with an oxidizing agent to produce (1,7 cyclic) Mpa-Har-Gly-Asp-Trp-Pro-Cys(Acm)-NH₂ and
e) purifying the product of step (d) by HPLC.

Yet another embodiment of the present invention provides a process for preparing desmopressin comprising the steps of:
a) providing a protected linear peptide having the formula Mpa(X)-Tyr(X)-Phe-Gln(X)-Asn(X)-Cys(Acm)-Pro-D-Arg(X)-Gly, wherein X is same or different protecting groups;
b) reacting the linear peptide of step (a) with an acidic composition to produce Mpa-Tyr-Phe-Gln-Asn-Cys(Acm)-Pro-D-Arg-Gly-NH₂
c) purifying the product of step (b) by HPLC;
d) treating the resulting linear peptide of step (c) with an oxidizing agent to produce (1,6 cyclic) Mpa-Tyr-Phe-Gln-Asn-Cys(Acm)-Pro-D-Arg-Gly-NH₂ and
e) purifying the product of step (d) by HPLC.

Another embodiment of the present invention provides a process for preparing calcitonin (salmon) comprising the steps of:
a) providing a protected linear peptide having the formula Cys(X)-Ser(X)-Asn(X)-Leu-Ser(X)-Thr(X)-Cys(Acm)-Val-Leu-Gly-Lys(X)-Leu-Ser(X)-Gm(X)-Glu(X)-Leu-His(X)-Lys(X)-Leu-Gln(X)-Thr(X)-Tyr(X)-Pro-Arg(X)-Thr(X)-Asn(X)-Thr(X)-Gly-Ser(X)-Gly-Thr(X)-Pro wherein X is same or different protecting groups;
b) reacting the linear peptide of step (a) with an acidic composition to produce Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂;
c) purifying the product of step (b) by HPLC;
d) treating the resulting linear peptide of step (c) with an oxidizing agent to produce (1,7 cyclic) Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂; and
e) purifying the product of step (d) by HPLC.

While the present invention is described with respect to particular examples and preferred embodiments, it is understood that the present invention is not limited to these examples and embodiments. The present invention as claimed therefore includes variations from the particular examples and preferred embodiments described herein, as will be apparent to one of skill in the art.

### Examples

### Example 1: Preparation of H-D-Phe-Cys-Phe-D-Trp-Lys-Thr-Cys(Acm)-Thr-ol (SEQ. ID NO.1)

Synthesis of the peptide was carried out by a stepwise Fmoc SPPS (solid phase peptide synthesis) procedure starting from Thr(t-Bu)-ol-2-Cl-Trt resin (100 g, loading of 0.7 mmol on 1 g of preloaded resin). After washing of the resin the second amino acid (Fmoc-Cys(Acm)) was introduced to start the first coupling step. Fmoc protected amino acid was activated in situ using TBTU/HOBt (N-hydroxybenzotriazole) and subsequently coupled to the resin for 50 minutes. Diisopropylethylamine was used during coupling as an organic base. Completion of the coupling was indicated by Ninhydrine test. After washing of the resin, the Fmoc protecting group on the ∀-amine was removed with 20% piperidine in DMF for 20 min. These steps were repeated each time with another amino acid according to peptide sequence. All amino acids used were Fmoc-N^{∀} protected except the last amino acid in the sequence, Boc-D-Phe. Trifunctional amino acids were side chain protected as follows: Thr(t-Bu), Cys(Trt), Cys(Acm), and Lys(Boc). Three equivalents of the activated amino acids were employed in the coupling reactions. At the end of the synthesis the peptide-resin was washed with DMF, followed by DCM, and dried under vacuum to obtain 223 g dry peptide-resin.

The peptide, prepared as described above, was cleaved from the resin using a 95% TFA, 2.5% TIS, 2.5% EDT solution for 2 hours at room temperature. The product was precipitated by the addition of 10 volumes of ether, filtered and dried in vacuum to obtain 111.7 g powder. It was identified by LC/MS as H-D-Phe-Cys-Phe-D-Trp-Lys-Thr-Cys(Acm)-Thr-ol.

### Example 2: Preparation of Octreotide

D-Phe—Cys—Phe—D-Trp—Lys—Thr—Cys—Thr-ol

H-D-Phe-Cys-Phe-D-Trp-Lys-Thr-Cys(Acm)-Thr-ol (SEQ. ID NO. 1) crude peptide (100 g, prepared as described in example (1) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing octreotide trifluoroacetate at a purity of >98.5%. After treatment to replace trifluoroacetate, the fractions were collected and lyophilized to obtain final dry peptide. The yield was 33 g (>98.5% pure).

### Example 3: Preparation of Mpa-Har-Gly-Asp-Trp--Cys(Acm)-NH₂ (Eptifibatide precursor)

Synthesis of the peptide was carried out by a regular stepwise Fmoc SPPS (solid phase peptide synthesis) procedure starting from 2-Cl-Trt resin (50 g). The first amino acid (Fmoc-Cys(Acm)) was loaded onto the resin in a preliminary step to provide loading of about 0.7 mmol/g. After resin washing, a second amino acid (Fmoc-Pro) was introduced to start the first coupling step. Fmoc protected amino acid was activated in situ using TBTU/HOBt and subsequently coupled to the resin for 50 minutes. Diisopropylethylamine or Collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected except the last building block in the sequence, Trt-Mpa Trifunctional amino acids were side chain protected as follows: Asp(tBu), Har(Pbf), and Cys(Acm). Three equivalents of the activated amino acids were employed in the coupling reactions. At the end of the synthesis the peptide-resin was washed with DMF, followed by DCM, and dried under vacuum to obtain 80 g dry peptide-resin.

The peptide, prepared as described above, was cleaved from the resin by washing with a solution of 1% TFA in DCM. The resulted solution was neutralized by addition of DIPEA and concentrated to about 10% peptide content. Amidation of the C-terminus was achieved by activation of the carboxy terminus with DCC/HOBt and coupling with ammonia solution in IPA. After removal of the solvent the protected peptide was precipitated in ether and dried. The protecting groups were removed using a 95% TFA, 2.5% TIS, 2.5% EDT solution for 2 hours at room temperature. The product was precipitated by the addition of 10 volumes of ether, filtered and dried in vacuum to obtain 30 g product.

### Example 4: Preparation of Eptifibatide

Mpa—Har—Gly—Asp—Trp—Pro—Cys—NH2

Mpa-Har-Gly-Asp-Trp-Pro-Cys(Acm)-NH₂ crude peptide (30 g, prepared as described in example 3) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing eptifibatide trifluoroacetate at a purity of >98.5%. The fractions were collected and lyophilized to obtain final dry peptide 6.9 g (>98.5% pure).

### Example 5: Preparation of Mpa-Tyr-Phe-Gln-Asn-Cys(Acm)-Pro-D-Arg-Gly-NH₂ (Desmopressin precursor) by SPPS method

Synthesis of the peptide was carried out by a regular stepwise Fmoc SPPS procedure starting from Rink amide resin (50 g). The first amino acid (Fmoc-Gly) was loaded on the resin by a regular coupling procedure after removal of the Fmoc group from the resin. After washing of the resin the second amino acid (Fmoc-D-Arg(Pbf)) was introduced to continue sequence elongation. Fmoc protected amino acids were activated in situ using TBTU/HOBt and subsequently coupled to the resin over about 50 minutes. Diisopropylethylamine or collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected except the last building block in the sequence, Trt-Mpa. Trifunctional amino acids were side chain protected as follows: Gln(Trt), D-Arg(Pbf), Tyr(tBu) and Cys(Acm). Three equivalents of the activated amino acids were employed in the coupling reactions. At the end of the synthesis the peptide-resin was washed with DMF, followed by DCM, and dried under vacuum to obtain 91 g dry peptide-resin.

The peptide, prepared as described above, was cleaved from the resin using a 89% TFA, 5.0% Phenol, 1.0% TIS, 2.5% EDT, 2.5% water solution for 1.5 hours at room temperature. The product was precipitated by the addition of 10 volumes of ether, filtered and dried in vacuum to obtain 49.0 g powder. It was identified by LC/MS as Mpa-Tyr-Phe-Gln-Asn-Cys(Acm)-Pro-D-Arg-Gly-NH₂.

### Example 6: Preparation of Mpa-Tyr-Phe-Gln-Asn-Cys(Acm)-Pro-D-Arg-Gly-NH₂ (Desmopressin precursor) in solution method

Synthesis of the peptide is carried out by a regular stepwise "solution synthesis" method. The second amino acid (Fmoc-D-Arg(Pbf)-OH) is dissolved in DMF and preactivated by addition of TBTU/HOBt in the presence of DIPEA. The first amino acid (Gly-NH₂) is dissolved in DMF, is added, and the reaction continues for about 1 h at room temperature. DMF is removed under low pressure and the residue is dissolved in ethylacetate. The organic solution is washed several times with aqueous HCl (1N), water and, NaHCO₃ (5%). After the solution is dried over Na₂SO₄, the solvent is evaporated to obtain Fmoc-D-Arg(Pbf)-Gly-NH₂. Fmoc group is removed by dissolution in piperidine/DMF (20%). The solution is concentrated and the crude di-peptide is precipitated in cold ether. By a similar procedure the rest of amino acids are added sequentially to obtain final protected linear peptide. Fmoc protected amino acids are activated in situ using TBTU/HOBt and subsequently coupled to the growing peptide chain. Diisopropylethylamine or collidine are used during coupling as an organic base. Completion of the coupling is determined by HPLC or TLC test. These steps are repeated each time with another amino acid according to the peptide sequence. All amino acids used are Fmoc-N^{α} protected except the last building block in the sequence, Trt-Mpa. Trifunctional amino acids are side chain protected as follows: Gln(Trt), D-Arg(Pbf), Tyr(tBu) and Cys(Acm).

The peptide, prepared as described above, is deprotected from its acid-labile protecting groups using a 91.5% TFA, 1.0% TIS, 2.5% EDT, 5.0% water solution for 1.5 hours at room temperature. The crude product, Mpa-Tyr-Phe-Gln-Asn-Cys(Acm)-Pro-D-Arg-Gly-NH₂ is precipitated by the addition of 10 volumes of ether, filtered, and dried in a vacuum to obtain fine powder. The product is identified by LC/MS.

### Example 7: Preparation ofDesmopressin

Mpa—Tyr—Phe—Gln—Asn—Cys—Pro—D-Arg—Gly—NH2

Mpa-Tyr-Phe-Gln-Asn-Cys(Acm)-Pro-D-Arg-Gly-NH₂ (SEQ. ID NO. 3) crude peptide (49 g, prepared as described in example 5) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing desmopressin trifluoroacetate at a purity of >98.5%. After exchange of the counterion to acetate the fractions were collected and lyophilized to obtain final dry peptide 14.9 g (>99.0% pure).

### Example 8: Preparation of Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂. (Calcitonin salmon precursor)

Synthesis of the peptide was carried out by a regular stepwise Fmoc SPPS procedure starting from Rink amide resin (30 g). The first amino acid (Fmoc-Pro) was loaded on the resin by a regular coupling procedure after removal of the Fmoc group from the resin. After washing of the resin the second amino acid (Fmoc-Thr(tBu)) was introduced to continue sequence elongation. Fmoc protected amino acids were activated in situ using TBTU/HOBt and subsequently coupled to the resin during about 60 minutes. Diisopropylethylamine or collidine were used during coupling as an organic base. Completion of the coupling was indicated by ninhydrine test. After washing of the resin, the Fmoc protecting group on the α-amine was removed with 20% piperidine in DMF for 20 min. These steps were repeated each time with another amino acid according to peptide sequence. All amino acids used were Fmoc-N^{α} protected. Trifunctional amino acids were side chain protected as follows: Cys(Trt), Ser(tBu), Asn(Trt), Gln(Trt), Thr(tBu), Glu(tBu), His(Trt), Lys(Boc), Arg(Pbf), Tyr(tBu) and Cys(Acm). Three equivalents of the activated amino acids were employed in the coupling reactions. At the end of the synthesis the peptide-resin was washed with DMF, followed by DCM, and dried under vacuum to obtain 77 g dry peptide-resin.

The peptide, prepared as described above, was cleaved from the resin using a 94% TFA, 1.0% TIS, 2.5% EDT, 2.5% water solution for 1.5 hours at room temperature. The product was precipitated by the addition of 10 volumes of ether, filtered and dried in vacuum to obtain 42.0 g powder. It was identified by LC/MS as Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂.

### Example 9: Preparation of Calcitonin (salmon)

Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂ crude peptide (42 g, prepared as described in example 7) was purified on preparative C₁₈ RP-HPLC column. Fractions containing >95% pure product were combined and diluted to concentrations of about 1 g/L. An equimolar amount of iodine in acetic acid was added under vigorous mixing at room temperature and subsequently excess iodine was neutralized by small amount of ascorbic acid. The resulting solution was loaded on a C₁₈ RP-HPLC column and purified to obtain fractions containing calcitonin trifluoroacetate at a purity of >98.5%. After exchange of the counterion to acetate the fractions were collected and lyophilized to obtain final dry peptide 8.8 g (>99.0% pure). The analytical method for reverse-phase chromatography (HPLC) used a 5µ C18 column, 0.05% TFA in acetonitrile/water eluent, and a flow rate of 1 ml/min.

## Claims

1. A process for preparing cyclic peptides comprising the steps of:
a) providing a protected linear peptide containing at least two protected thiol-containing residues of which at least one thiol-containing residue is protected with an orthogonal protecting group;
b) reacting the protected linear peptide with an acidic composition to produce a semi-protected linear peptide with the orthogonal protecting group on one of the thiol-containing residues;
c) purifying the semi-protected linear peptide;
d) treating the purified semi-protected linear peptide obtained in step (c) with an oxidizing agent to produce a cyclic peptide; and
e) purifying the cyclic peptide to obtain a purified cyclic peptide.

2. The process of claim 1, wherein the cyclic peptide is selected from the group consisting of somatostatin analogues, vasopressin related peptides, α-atrial natriuretic factors/peptides (ANF/ANP), calcitonins, and other disulfide containing peptides.

3. The process of claim 1, wherein the cyclic peptide is selected from the group consisting of octreotide, calcitonin (salmon), desmapressin, oxytocin, nesiritide, and eptifibatide.

4. The process of claim 1, wherein the protected linear peptide provided in step (a) is attached to a resin.

5. The process of claim 1, wherein the purified cyclic peptide of step (e) has a purity of at least about 98.5% by HPLC.

6. The process of claim 1, wherein the purified cyclic peptide of step (e) has a purity of at least about 99% by HPLC.

7. The process of claim 1, wherein the orthogonal protecting group is a non-acid labile protecting group selected from the group consisting of acetamidomethyl, benzyl, 4-methoxybenzyl, tert-butyl, trimethylacetamidomethyl, phenylacetamidomethyl, and tert-butylmercapto.

8. The process of claim 7, wherein the non-acid labile protecting group is acetamidomethyl.

9. The process of claim 1, further comprising neutralizing excess oxidizing agent after step (d).

10. The process of claim 1, further comprising drying the purified cyclic peptide.

11. The process of claim 1, wherein the acidic composition comprises trifluoroacetic acid.

12. The process of claim 11, wherein the acidic composition further comprises triisopropylsilane and ethanedithiol.

13. The process of claim 1, wherein the oxidizing agent is iodine.

14. The process of claim 1, wherein the purification steps (d) and (e) are carried out using HPLC.

15. The process of claim 1 comprising the steps of
a) providing a protected linear peptide having the formula X-D-Phe-Cys(X)-Phe-D-Trp-Lys(X)-Thr(X)-Cys-(Acm)-Thr(X)-ol, wherein X is a same or different protecting group;
b) reacting the protected linear peptide of step (a) with an acidic composition to produce a semi-protected linear peptide D-Phe-Cys-Phe-D-Trp-Lys-Thr-Cys(Acm)-Thr-ol;
c) purifying the semi-protected linear peptide of step (b) using HPLC;
d) treating the purified semi-protected linear peptide of step (c) with an oxidizing agent to produce a cyclic peptide (2,7 cyclic) D-Phe-Cys-Phe-D-Trp-Lys-Thr-Cys-Thr-ol and
o) purifying the cyclic peptide of step (d) using HPLC to obtain a purified cyclic peptide.

16. The process of claim 1 comprising the steps of:
a) providing a protected linear peptide having the formula Mpa(X)-Har(X)-Gly-Asp(X)-Trp-Pro-Cys(Acm) wherein X is a same or different protecting group;
b) reacting the protected linear peptide of step (a) with an acidic composition to produce a semi-protected linear peptide Mpa-Har-Gly-Asp- Trp-Pro-Cys(Acm)NH₂
c) purifying the semi-proteoted linear peptide of step (b) using HPLC;
d) treating the purified semi-protected linear peptide of stop (c) with an oxidizing agent to produce a cyclic peptide (1,7 cyclic) Mpa-Har-Gly-Asp-Trp-Pro-Cys(Acm)-NH₂; and
e) purifying the cyclic peptide of step (d) using HPLC to obtain a purified cyclic peptide.

17. The process of claim 1 comprising the steps of:
a) providing a protected linear peptide having the formula Mpa(X)-Tyr(X)-Phe-Gln(X) Asn(X)-Cys(Acm)-Pro-D-Arg(X)-Gly wherein X is a same or different protecting group;
b) reacting the protected linear peptide of step (a) with an acidic composition to produce a semi-protected linear peptide Mpa-Tyr-Phe-Gln-Asn-Cys(Acm)-Pro-D-Arg-Gly-NH₂;
c) purifying the semi-protected linear peptide of step (b) using HPLC;
d) treating the purified semi-protected linear peptide of step (c) with an oxidizing agent to produce a cyclic peptide (1,6 cyclic) Mpa-Tyr-Phe-Gln-Asn-Cys(Acm)-Pro-D-Arg-Gly-NH₂ and
e) purifying the cyclic peptide of step (d) using HPLC to obtain a purified cyclic peptide.

18. The process of claim 1 comprising the steps of:
a) providing a protected linear peptide having the formula Cys(X)-Ser(X)-Asn(X)-Leu, Ser(X)-Thr(X)-Cys(Acm)-Val-Leu-Gly-Lys(X)-Leu-Ser(X)-Gln(X)-Glu(X)-Leu-His(X)-Lys(X)-Leu-Gln(X)-Thr(X)-Tyr(X)-Pro-Arg(X)-Thr(X)-Asn(X)-Thr(X)-Gly-Ser(X)-Gly-Thr(X)-Pro-NH₂ wherein X is a same or different protecting group;
b) reacting the protected linear peptide of step (a) with an acidic composition to produce a semi-protected linear peptide Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂;
c) purifying the semi-protected linear peptide of step (b) using HPLC;
d) treating the purified semi-protected linear peptide of step (o) with an oxidizing agent to produce a cyclic peptide (1,7 cyclic) Cys-Ser-Asn-Leu-Ser-Thr-Cys(Acm)-Val-Leu-Gly-Lys Leu-Ser-Gla-Glu-Leu-His-Lys-Leu-Gln-Thr-Tyr-Pro-Arg-Thr-Asn-Thr-Gly-Ser-Gly-Thr-Pro-NH₂ and
e) purifying the cyclic peptide of step (d) using HPLC to obtain a purified cyclic peptide.

19. The process of claim 15, 16,17 or 18, wherein the purified cyclic peptide of step (e) has a purity of at least about 98.5% by HPLC.

20. The process of claim 15,16,17 or 18, wherein the purified cyclic peptide of step (e) has a purity of at least about 99% by HPLC.

## Patentansprüche

1. Verfahren zur Herstellung zyklischer Peptide, welches die folgenden Stufen umfaßt:
a) Bereitstellen eines geschützten linearen Peptids, welches wenigstens zwei geschützte, Thiol enthaltende Reste enthält; von denen wenigstens ein Thiol enthaltender Rest mit einer orthogonalen Schutzgruppe geschützt ist,
b) Umsetzen des geschützten linearen Peptids mit einer sauren Zusammensetzung unter Erzeugung eines halb-geschützten linearen Peptids mit der orthogonalen Schutzgruppe auf einem der Thiol enthaltenden Reste,
c) Reinigen des halb-geschützten linearen Peptids,
d) Behandeln des in Stufe (c) erhaltenen gereinigten, halb-geschützten linearen Peptids mit einem Oxidationsmittel unter Erzeugung eines zyklischen Peptids und
e) Reinigen des zyklischen Peptids unter Erhalt eines gereinigten zyklischen Peptids.

2. Verfahren nach Anspruch 1, wobei das zyklische Peptid aus der Gruppe ausgewählt ist, bestehend aus Somatostatinanalogen, mit Vasopressin verwandten Peptiden, α-atrialen natriuretischen Faktoren/Peptiden (ANF/ANP). Calcitoninen und anderen Disulfid enthaltenden Peptiden.

3. Verfahren nach Anspruch 1, wobei das zyklische Peptid aus der Gruppe ausgewählt ist, bestehend aus Octreotid, Calcitonin (Lachs), Desmopressin, Oxytocin, Nesiritid und Eptifibatid.

4. Verfahren nach Anspruch 1, wobei das in Stufe (a) bereitgestellte geschützte lineare Peptid an ein Harz angebunden ist.

5. Verfahren nach Anspruch 1, wobei das gereinigte zyklische Peptid aus Stufe (e) eine Reinheit von wenigstens etwa 90,5%, ermittelt durch HPLC, hat.

6. Verfahren nach Anspruch 1, wobei das gereinigte zyklische Peptid aus Stufe (e) eine Reinheit von wenigstens etwa 99%, ermittelt durch HPLC. hat.

7. Verfahren nach Anspruch 1, wobei die orthogonale Schutzgruppe eine nicht-saure, labile Schutzgruppe ist, ausgewählt aus der Gruppe, bestehend aus Acetamidomethyl, Benzyl, 4-Methoxybenzyl, tert-Butyl, Trimethylacetamidomethyl, Phenylacetamidomethyl und tert-Butylimercapto.

8. Verfahren nach Anspruch 7, wobei die nicht-saure, labile Schutzgruppe Acetamidomethyl ist.

9. Verfahren nach Anspruch 1, welches weiterhin das Neutralisieren von überschüssigem Oxidationsmittel nach Stufe (d) umfaßt.

10. Verfahren nach Anspruch 1, welches weiterhin das Trocknen des gereinigten zyklischen Peptids umfaßt.

11. Verfahren nach Anspruch 1, wobei die saure Zusammensetzung Trifluoressigsäure umfaßt.

12. Vorfahren nach Anspruch 11, wobei die saure Zusammensetzung weiterhin Triisopropylsilan und Ethandithiol umfaßt.

13. Verfahren nach Anspruch 1, wobei das Oxidationsmittel lod ist.

14. Verfahren nach Anspruch 1, wobei die Reinigungsstufen (d) und (e) unter Verwendung von HPLC ausgeführt werden.

15. Verfahren nach Anspruch 1, welches die folgenden Stufen umfaßt:
a) Bereitstellen eines geschützten linearen Peptids der Formel X-D-Phe-Cys(X)-Phe-D-Trp-Lys(X)- Thr(X)-Cys(Acm)-Thr(X)-ol, wobei X gleiche oder verschiedene Schutzgruppen bedeutet,
b) Umsetzen des geschützten linearen Peptids aus Stufe (a) mit einer sauren Zusammensetzung unter Erzeugung eines halb-geschützten linearen Peptids D-Phe-Cys-Phe-D-Trp-Lys-Thr-Cys(Acm)-Thr-ol,
c) Reinigen des halb-geschützten linearen Peptids aus Stufe (b) unter Verwendung von HPLC,
d) Behandeln des gereinigten, halb-geschützten linearen Peptids aus Stufe (c) mit einem Oxidationsmittel unter Erzeugung eines zyklischen Peptids (2,7-zyklisches) D-Phe-Cys-Phe-D-Trp-Lys-Thr-Cys-Thr-ol und
e) Reinigen des zyklischen Peptids aus Stufe (d) unter Verwendung von HPLC unter Erhalt eines gereinigten zyklischen Peptids.

16. Verfahren nach Anspruch 1, welches die folgenden Stufen umfaßt:
a) Bereitstellen eines geschützten linearen Peptids der Formel Mpa(X)-Har(X)-Gly-Asp(X)-Trp-Pro-Cys(Acm), wobei X gleiche oder verschiedene Schutzgruppen bedeutet.
b) Umsetzen des geschützten linearen Peptids aus Stufe (a) mit einer sauren Zusammensetzung unter Erzeugung eines halb-geschützten linearen Peptids Mpa-Har-Gly-Asp-Trp-Pro-Cys(Acm)-NH₂,
c) Reinigen des halb-geschützten linearen Peptids aus Stufe (b) unter Verwendung von HPLC,
d) Behandeln des gereinigten, halb-geschützten linearen Peptids aus Stufe (c) mit einem Oxidationsmittel unter Erzeugung eines zyklischen Peptids (1,7-zyklisches) Mpa-Har-Gly-Asp-Trp-Pro-Cys(Acm)-NH₂ und
e) Reinigen des zyklischen Peptids aus Stufe (d) unter Verwendung von HPLC unter Erhalt eines gereinigten zyklischen Peptids.

17. Verfahren nach Anspruch 1, welches die folgenden Stufen umfaßt:
a) Bereitstellen eines geschützten linearen Peptids der Formel Mpa(X)-Tyr(X)-Phe-Gln(X)-Asn(X)-Cys(Acm)-Pro-D-Arg(X)-Gly, wobei X gleiche oder verschiedene Schutzgruppen bedeutet,
b) Umsetzen des geschützten linearen Peptids aus Stufe (a) mit einer sauren Zusammensetzung unter Erzeugung eines halb-geschützten linearen Peptids Mpa-Tyr-Phe-Gln-Asn-Cys(Acm)-Pro-D-Arg-Gly-NH₂,
c) Reinigen des halb-geschützten linearen Peptids aus Stufe (b) unter Verwendung von HPLC.
d) Behandeln des gereinigten, halb-geschützten linearen Peptids aus Stufe (c) mit einem Oxidationsmittel unter Erzeugung eines zyklischen Peptids (1,6-zyklisches) Mpa-Tyr-Phe-Gln-Asn-Cys(Acm)-Pro-D-Arg-Gly-NH₂ und
e) Reinigen des zyklischen Peptids aus Stufe (d) unter Verwendung von HPLC unter Erhalt eines gereinigten zyklischen Peptids.

18. Verfahren nach Anspruch 1, welches die folgenden Stufen umfaßt:
a) Bereitstellen eines geschützten linearen Peptids der Formel Cys(X)-Ser(X)-Asn(X)-Leu-Ser(X)-Thr(X)-Cys(Acm)-Val-Leu-Gly-Lys(X)-Leu-Ser(XyGln(X)-Glu(X)-Leu-His(X)-Lys(X)-Leu-Gln(X)-Thr(X)-Tyr(X)-Pro-Arg(X)-Thr(X)-Asn(X)-Thr(X)-Gly-Ser(X)-Gly-Thr(X)-Pro-NH₂, wobei X gleiche oder verschiedene Schutzgruppen bedeutet,
b) Umsetzen des geschützten linearen Peptids aus Stufe (a) mit einer sauren Zusammensetzung unter Erzeugung eines halb-geschützten linearen Peptids Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂.
c) Reinigen des halb-geschützten linearen Peptids aus Stufe (b) unter Verwendung von HPLC,
d) Behandeln des gereinigten, halb-geschützten linearen Peptids aus Stufe (c) mit einem Oxidationsmittel unter Erzeugung eines zyklischen Peptids (1,7-zyklisches) Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr Gly-Ser-Gly-Thr-Pro-NH₂ und
e) Reinigen des zyklischen Peptids aus Stufe (d) unter Verwendung von HPLC unter Erhalt eines gereinigten zyklischen Peptids.

19. Verfahren nach einem der Ansprüche 15, 16, 17 oder 18, wobei das gereinigte zyklische Peptid aus Stufe (e) eine Reinheit von wenigstens etwa 98,5%, ermittelt durch HPLC, hat.

20. Verfahren nach einem der Ansprüche 15, 16, 17 oder 18, wobei das gereinigte zyklische Peptid aus Stufe (e) eine Reinheit von wenigstens etwa 99%, ermittelt durch HPLC, hat.

## Revendications

1. Processus de préparation de peptides cycliques comprenant les étapes consistant à :
a) fournir un peptide linéaire protégé contenant au moins deux résidus protégés contenant du thiol desquels au moins un résidu contenant du thiol est protégé avec un groupement protecteur orthogonal ;
b) faire réagir le peptide linéaire protégé avec une composition acide pour produire un peptide linéaire semi-protégé avec le groupement protecteur orthogonal sur un des résidus contenant du thiol ;
c) purifier le peptide linéaire semi-protégé ;
d) traiter le peptide linéaire semi-protégé purifié obtenu dans l'étape (c) avec un agent oxydant pour produire un peptide cyclique ; et
e) purifier le peptide cyclique pour obtenir un peptide cyclique purifié.

2. Processus selon la revendication 1, dans lequel le peptide cyclique est choisi dans le groupe comprenant les analogues de somatostatine, peptides parents de la vasopressine, facteurs/peptides natriurétiques α-atriaux (ANF/ANP) calcitonines, et autres peptides contenant un disulfure.

3. Processus selon la revendication 1, dans lequel le peptide cyclique est choisi dans le groupe comprenant les octréotide, calcitonine (saumon), desmopressine, oxytocine, nésiritide, et eptifibatide.

4. Processus selon la revendication 1, dans lequel le peptide linéaire protégé fourni dans l'étape (a) est attaché à une résine.

5. Processus selon la revendication 1, dans lequel le peptide cyclique purifié de l'étape (c) présente une pureté d'au moins environ 98,5 % par HPLC.

6. Processus selon la revendication 1, dans lequel le peptide cyclique purifié de l'étape (e) présente une pureté d'au moins environ 99 % par HPLC.

7. Processus selon la revendication 1, dans lequel le groupement protecteur orthogonal est un groupement protecteur labile non acide choisi dans le groupe comprenant les acétamidométhyle, benzyle, 4-méthoxybenzyle, tert-butyle, triméthylacétamidométhyle, phénylacétamidométhyle et tert-butylmercapto.

8. Processus selon la revendication 7, dans lequel le groupement protecteur labile non acide est l'acétamidométhyle.

9. Processus selon la revendication 1, comprenant en outre la neutralisation de l'excès d'agent oxydant après l'étape (d).

10. Processus selon la revendication 1, comprenant en outre le séchage du peptide cyclique purifié.

11. Processus selon la revendication 1, dans lequel la composition acide comprend acide trifluoroacétique.

12. Processus selon la revendication 11, dans lequel la composition acide comprend en outre triisopropylsilane et éthanedithiol.

13. Processus selon la revendication 1, dans lequel l'agent oxydant est l'iode.

14. Processus selon la revendication 1, dans lequel les étapes de purification (d) et (e) sont réalisées en utilisant la HPLC.

15. Processus selon la revendication 1 comprenant les étapes consistant à :
a) fournir un peptide linéaire protégé ayant la formule X-D-Phe-Cys(X)-Phe-D-Trp-Lys(X)-Thr(X)-Cys(Acm)-Thr(X)-ol, dans laquelle X est un groupement protecteur identique ou différent ;
b) faire réagir le peptide linéaire protégé de l'étape (a) avec une composition acide pour produire un peptide linéaire semi-protégé D-Phe-Cys-Phe-D-Trp-Lys-Thr-Cys(Acm)-Thr-ol ;
c) purifier le peptide linéaire semi-protégé de l'étape (b) en utilisant la HPLC ;
d) traiter le peptide linéaire semi-protégé purifié de l'étape (c) avec un agent oxydant pour produire un peptide cyclique (2,7 cyclique)
D-Phe-Cys-Phe-D-Trp-Lys-Thr-Cys-Thr-ol
et
e) purifier le peptide cyclique de l'étape (d) en utilisant la HPLC pour obtenir un peptide cyclique purifié.

16. Processus selon la revendication 1 comprenant les étapes consistant à :
a) fournir un peptide linéaire protégé ayant la formule
Mpa(X)-Har(X)-GlyAsp(X)-Trp-Pro-Cys(Acm),
dans laquelle X est un groupement protecteur identique ou différent ;
b) faire réagir le peptide linéaire protégé de l'étape (a) avec une composition acide pour produire un peptide linéaire semi-protégé Mpa-Har-GlyAsp-Trp-Pro-Cys(Acm)-NH₂ ;
c) purifier le peptide linéaire semi-protégé de l'étape (b) en utilisant la HPLC ;
d) traiter le peptide linéaire semi-protégé purifié de l'étape (c) avec un agent oxydant pour produire un peptide cyclique (1,7 cyclique)
Mpa-Har-Gly-Asp-Trp-Pro-Cys(Acm)-NH₂
et
e) purifier le peptide cyclique de l'étape (d) en utilisant la HPLC pour obtenir un peptide cyclique purifié.

17. Processus selon la revendication 1 comprenant les étapes consistant à :
a) fournir un peptide linéaire protégé ayant la formule
Mpa(X)-Tyr(X)-Phe-Gln(X)-Asn(X)-Cys(Acm)-Pro-D-Arg(X)-Gly,
dans laquelle X est un groupement protecteur identique ou différent ;
b) faire réagir le peptide linéaire protégé de l'étape (a) avec une composition acide pour produire un peptide linéaire semi-protégé
Mpa-Tyr-Phe-Gln-Asn-Cys(Acm)-Pro-D-Arg-Gly-NH₂;
c) purifier le peptide linéaire semi-protégé de l'étape (b) en utilisant la HPLC ;
d) traiter le peptide linéaire semi-protégé purifié de l'étape (c) avec un agent oxydant pour produire un peptide cyclique (1,6 cyclique)
Mpa-Tyr-Phe-GIn-Asn-Cys(Acm)-Pro-D-Arg-Gly-NH₂
et
e) purifier le peptide cyclique de l'étape (d) en utilisant la HPLC pour obtenir un peptide cyclique purifié.

18. Processus selon la revendication 1 comprenant les étapes consistant à :
a) fournir un peptide linéaire protégé ayant la formule
Cys(X)-Ser(X)-Asn(X)-Leu-Ser(X)-Thr(X)-Cys(Acm)-Val-Leu-Gly-Lys(X)-Leu-Ser(X)-Gln(X)-Glu(X)-Leu-His(X)-Lys(X)-Leu-Gln(X)-Thr(X)-Tyr(X) Pro-Arg(X)-Thr(X)-Asn(X)-Thr(X)-Gly-S er(X)-Gly-Thr(X)-Pro-NH₂,
dans laquelle X est un groupement protecteur identique ou différent ;
b) faire réagir le peptide linéaire protégé de l'étape (a) avec une composition acide pour produire un peptide linéaire semi-protégé
Cys-Ser-Asn-Leu-Ser-Thr-Cys(Acm)-Val-Leu-Gly-Lys-Leu-Ser-Gln-Glu-Leu His-Lys-Leu-Gln-Thr-Tyr-Pro-Arg-Thr-Asn-Thr-Gly-Ser-Gly-Thr-Pro-NH₂;
c) purifier le peptide linéaire semi-protégé de l'étape (b) en utilisant la HPLC ;
d) traiter le peptide linéaire semi-protégé purifié de l'étape (c) avec un agent oxydant pour produire un peptide cyclique (1,7 cyclique)
Cys-Ser-Asn-Leu-Ser-Thr-Cys(Acm)-Val-Leu-Gly-Lys-Leu-Ser-Gln-Glu-Leu-His-Lys-Leu-Gln-Thr-Tyr-Pro-Arg-Thr-Asn-Thr-Gly-Ser-Gly-Thr-Pro-NH₂
et
e) purifier le peptide cyclique de l'étape (d) en utilisant la HPLC pour obtenir un peptide cyclique purifié.

19. Processus selon la revendication 15, 16, 17 ou 18, dans lequel le peptide cyclique purifié de l'étape (e) présente une pureté d'au moins environ 98,5 % par HPLC.

20. Processus selon la revendication 15, 16, 17 ou 18, dans lequel le peptide cyclique purifié de l'étape (e) présente une pureté d'au moins environ 99 % par HPLC.
